# EUROPEAN PATENT SPECIFICATION

(11) **EP 2 036 446 B1**
(45) Date of publication and mention of the grant of the patent: **25.12.2013**
(21) Application number: 07790213.8
(22) Date of filing: 26.06.2007
(51) Int. Cl.: A23L 2/00, A23F 3/16, A23L 2/38, A23L 2/54, A23L 2/58

(54) **CARBONATED BEVERAGE PACKED IN CONTAINER**
kOHLENSÄUREHALTIGES VERPACKTES GETRÄNK
BOISSON CARBONISÉE CONDITIONNÉE DANS UN RÉCIPIENT

(30) Priority: 04.07.2006 JP 2006184308; 20.03.2007 JP 2007071802
(43) Date of publication of application: 18.03.2009
(73) Proprietor: KAO CORPORATION, Chuo-ku Tokyo 103-8210 (JP)
(72) Inventor: FUKUDA, Masahiro, Tokyo 131-8501 (JP); TAKAHASHI, Hirokazu, Tokyo 131-8501 (JP); KUSAKA, Ryo, Tokyo 131-8501 (JP); ITAYA, Eri, Tokyo 131-8501 (JP)
(74) Representative: HOFFMANN EITLE
(86) International application number: PCT/JP2007/000690
(87) International publication number: WO 2008/004339

(56) References cited:
- WO-A1-2005/053415
- WO-A1-2005/060761
- CN-A- 1 981 587
- JP-A- 2005 058 208
- JP-A- 2006 136 204
- US-A- 4 946 701
- US-A1- 2006 099 318
- MIYAZAKI PREFECTURE FOODS DEVELOPMENT CENTER: RESEARCH REPORT OF MIYAZAKI PREFECTURE INDUSTRIAL TECHNOLOGY CENTER, no. 44, 1999, pages 111-114,

## Description

### Field of the Invention

The present invention relates to a packaged beverage containing non-polymer catechins in concentration.

### Background of the Invention

It is reported that catechins have effects such as cholesterol-suppressing effect and α-amylase activity inhibitory effect (Patent Documents 1 and 2). In order for such physiological effects to materialize, a large amount of catechins need to be digested and therefore, there has been a strong demand for the development of a technology making it possible for a high concentration of catechins to be incorporated in a beverage. Among such technologies is a method designed to incorporate molten catechins in a beverage by using a concentrate of a green-tea extract (Patent Documents 3 to 5).

However, a commercially available concentrate of a green-tea extract has strong astringency or bitterness stemming from components contained therein and does not give rise to a good sensation when passing through the throat, if no modification is made thereto. Accordingly, a beverage that is capable of reducing the stringency/bitterness typical of a beverage containing a high concentration of catechins has been desired whatever its technique, from the standpoint of the prolonged applicability to drinking needed to induce continued physiological effects of catechins.

In order to reduce the bitterness of a tea beverage, a method designed to incorporate cyclodextrin therein is reported (Patent Documents 6 to 11). Specifically, Patent Document 6 discloses a tea-extract-containing composition in which 2.5 parts or more by mass of cyclodextrin are contained relative to 1 part by dry mass of a tea extract; Patent Document 7 discloses a method of preparing a food or drink containing 1 mass% or less of catechins, 0.1 mass% or less of caffeine, and from 0.1 to 20.0 mass% of cyclodextrin, wherein a tea extract is reacted with steam activated carbon to remove caffeine by adsorbing it thereto; and Patent Documents 8 and 9 disclose their packaged beverages containing catechins and cyclodextrin, each in a predetermined amount. Patent Document 10 discloses a packaged tea beverage having reduced bitterness obtained by incorporating cluster dextrin in a high concentration of tea catechins. Patent Document 11 discloses a suppressing effect of cyclodextrin on the bitterness of a high concentration of catechins.
Patent Document 12 discloses a food composition having an improved taste by using, in combination, sucralose for masking an unpleasant odor/unpleasant taste and a sweetener composition having a good sweetness quality such as thaumatin, fructose, non-reducing disaccharide, sugar alcohol, raffinose, licorice extract, stevia extract, rhamnose or glycyrrhizin; a preservative and food composition having an improved taste quality; and an odoriferous composition having an improved flavor feeling.

WO 2005/053415 discloses a packed high-catechin beverage with added low-caffeine green tea extract. The beverage is not carbonated.

However, a large amount of cyclodextrin is necessary to reduce a bitter taste left behind when a heat sterilization treatment is carried out, by adding it to a packaged beverage having a high concentration of non-polymer catechins. Moreover, a large amount of cyclodextrin could impair the original taste of a beverage because of the intrinsic taste of cyclodextrin, so its amount has to be limited considerably. On the other hand, even if an artificial sweetener is further incorporated in a packaged beverage, such incorporation alone is still not enough to suppress the bitterness of the packaged beverage containing non-polymer catechins in a high concentration. It is also reported that a beverage is produced by adding citric acid and cane sugar to a green-tea extract and then blow a carbon dioxide gas into the resulting mixture, aimed at reducing bitterness/astringency, browning or retort odor which could arise as the green-tea extract is obtained at an increased extraction concentration; (Non-patent Document 1).
[Patent Document 1] JP-A-60-156614
[Patent Document 2] JP-A-03-133928
[Patent Document 3] JP-A-2002-142677
[Patent Document 4] JP-A-08-298930
[Patent Document 5] JP-A-08-109178
[Patent Document 6] JP-A-03-168046
[Patent Document 7] JP-A-10-4919
[Patent Document 8] JP-A-2002-238518
[Patent Document 9] JP-A-2004-129662
[Patent Document 10] JP-A-2004-159641
[Patent Document 11] JP-A-2004-254511
[Patent Document 12] JP-A-2000-24273
[Non-patent Document 1] Research Report of Miyazaki Prefecture Industrial Technology Center/Miyazaki Prefecture Foods Development Center, Heisei 11 nendo (fiscal 1999), No. 44, p111-114

### [Disclosure of Invention]

The present invention provides a packaged effervescent beverage with a calorie less than 40 Kcal/240 ml, having a purified product of green tea extract added thereto, comprising:
(A) from 0.08 to 0.5 mass% of non-polymer catechins,
(B) from 0.0001 to 20 mass% of (B) a sweetener, and
(C) a carbon dioxide gas, whose gas volume ranges from more than 0.5 vol.% to 4.0 vol.%,
   wherein:
(D) a non-epicatechin percentage in the non-polymer catechins is from 5 to 25 mass%,
   a percentage of (E) gallates in (A) the non-polymer catechins ([(E)/(A)] × 100) is from 5 to 55 mass%,
(F) the beverage has a pH of from 2.5 to 5.1; and
   a mass ratio of (G) caffeine to (A) the non-polymer catechins [(G)/(A)] is from 0.0001 to 0.16; and wherein the sweetener (B) comprises erythritol and at least one selected from fruit sugar, grape sugar and high-fructose corn syrup.

### Embodiments of the Invention

The present invention provides a packaged effervescent beverage containing a high concentration of catechins, which exhibits both reduced bitterness and adequate sweetness, and not undergoing a substantial reduction in the catechin content even after long storage albeit containing a sweetener.

The present inventors have carried out an investigation with a view to reducing bitterness without deteriorating the taste of a packaged beverage containing a high concentration of non-polymer catechins. As a result, it has been found that an excellent bitterness-suppressing effect can be achieved by incorporating a sweetener at a predetermined ratio and also incorporating a carbon dioxide gas in the beverage, and a reduction in the catechin content in the beverage can be minimized by controlling a non-epicatechin percentage in the non-polymer catechins, a pH, and a caffeine/non-polymer catechin ratio, so that it becomes possible to obtain a packaged effervescent beverage whose original taste continues to be preserved for a long period of time.

The present invention has made it possible to provide a packaged effervescent beverage containing non-polymer catechins, which has a good taste, has reduced bitterness and does not undergo a substantial reduction in the catechin content thereof even after long storage.

The term "non-polymer catechins" (A) as used herein is a generic term, which collectively encompasses non-epicatechins such as catechin, gallocatechin, catechin gallate, and gallocatechin gallate, and epicatechins such as epicatechin, epigallocatechin, epicatechin gallate, and epigallocatechin gallate. The concentration of the non-polymer catechins is determined based on the total mass of these eight non-polymer catechins.

The packaged effervescent beverage of the present invention has a non-polymer catechin content of from 0.08 to 0.5 mass%, preferably from 0.09 to 0.4 mass%, more preferably from 0.1 to 0.3 mass%, even more preferably from 0.1 to 0.2 mass%. The packaged effervescent beverage having a non-polymer catechin content within the above-described range facilitates digestion of a large amount of the non-polymer catechins and is therefore expected to produce sufficient physiological effect of the non-polymer catechins. When the non-polymer catechin content is less than 0.08 mass%, such a beverage does not produce the physiological effectsuffciently. When the non-polymer catechin content exceeds 0.5 mass%, on the other hand, such a beverage is not suited for drinking because of increased bitterness.

Non-epicatechins are rarely found in nature, and these catechins are normally obtained by thermal isomerization of epicatechins. Non-polymer catechins are converted into polymer catechins by thermal modification. The non-epicatechin percentage (D) in the non-polymer catechins (A) which is usable in the packaged effervescent beverage of the present invention is preferably from 5 to 25 mass%, more preferably from 8 to 20 mass%, even more preferably from 10 to 15 mass% from the viewpoints of the taste and storage stability of the non-polymer catechins.

The non-polymer catechins in the packaged effervescent beverage of the present invention can be classified into gallates such as epigallocatechin gallate, gallocatechin gallate, epicatechin gallate, and catechin gallate and non-gallates such as epigallocatechin, gallocatechin, epicatechin, and catechin. The gallates which are ester type non-polymer catechins have a strong bitter taste so that the percentage of (E)the gallates in (A)the non-polymer catechins ([(E)/(A)] × 100), which is usable in the present invention, is preferably from 5 to 55 mass%, more preferably from 8 to 46 mass% from the viewpoint of reducing the bitterness of the beverage. The concentration of the gallates in the non-polymer catechins in the present invention is preferably within a range of from 30 to 100 mg/100 ml, because it improves a refreshing taste after drinking.

The packaged effervescent beverage of the present invention having a high concentration of non-polymer catechins can be obtained, for example, by controlling the concentration of the non-polymer catechins with a purified product of green tea extract. More specifically, an aqueous solution of a purified product of green tea extract or a mixture of a green-tea extract in the purified product of green tea extract is usable. The purified product of green tea extract usable here is obtained by extracting green tea leaves with hot water or a water soluble organic solvent and then removing some portion of water from the resulting extracted solution or purifying the resulting extracted solution to raise the non-polymer catechin concentration thereof. It is provided in various forms such as solid, aqueous solution and slurry. The green-tea extract available from green tea leaves is an extracted solution not subjected to a concentration or purification operation.

Examples of commercially-available purified product of green tea extract containing non-polymer catechins include "Polyphenon" of Mitsui Norin Co., Ltd., "THEA-FLAN" of ITO EN Co., Ltd., and "Sunphenon" of Taiyo Kagaku Co., Ltd. These purified product of green tea extracts can be used after purification insofar as the resulting purified products have a non-polymer catechin concentration falling within the above-described range. Purification may be performed, for example, by suspending the purified product of green tea extract in water or a mixture of water and an organic solvent such as ethanol, removing the precipitate thus formed and then distilling off the solvent. Alternatively, products obtained by concentrating an extract of tea leaves with hot water or a water-soluble organic solvent such as ethanol and then purifying the concentrate or by purifying the extract directly may be used.

The gallate percentage of the non-polymer catechins used in the invention can be reduced by treating the purified product of green tea extract with tannase. The treatment with tannase is preferably performed by adding tannase in an amount to fall within a range of from 0.5 to 10 mass% relative to the precipitate of the green-tea extract or the non-polymer catechins in the precipitate. The treatment with tannase is performed at a temperature which does not affect the enzyme activity, preferably from 15 to 40°C, more preferably from 20 to 30°C. The treatment with tannase is performed at a pH which does not affect the enzyme activity, preferably from 4 to 6, more preferably from 4.5 to 6, far more preferably from 5 to 6.

The mass ratio of caffeine (G) to (A) the non-polymer catechins [(G)/(A)] in the packaged effervescent beverage of the present invention is preferably from 0.0001 to 0.16, more preferably from 0.001 to 0.15, still more preferably from 0.01 to 0.14, still more preferably from 0.05 to 0.13. The mass ratio [(G)/(A)] within the above-described range cannot be achieved when an extracted solution obtained by extraction of ordinary green tea leaves is used. In the packaged effervescent beverage of the present invention, the mass ratio [(G)/(A)] within the above-described range can be achieved by using the purified product of green tea extract containing non-polymer catechins. When the ratio of caffeine to the non-polymer catechins is unduly low, the resulting beverage is preferred from the viewpoint of a taste balance. When the ratio of caffeine to the non-polymer catechins is unduly high, on the other hand, the resulting beverage is not preferred because the original appearance thereof is impaired. Caffeine may be either caffeine existing naturally in a green-tea extract, flavor, juice or another component used as the raw material or caffeine added newly.

The packaged effervescent beverage of the present invention has a pH (F) falling within a range of preferably from 2.5 to 5.1, more preferably from 2.8 to 5.0, still more preferably from 3.0 to 4.5, even more preferably from 3.8 to 4.2 from the viewpoints of taste and storage stability. When the pH less than 2.5, the resulting beverage has an strong acid taste and the non-polymer catechin content of the beverage decreases during storage. When the pH exceeds 5.1, on the other hand, the non-polymer catechin content decreases during storage because of the reaction with a carbohydrate used in combination. The pH can be adjusted to fall within the above-described range by using ascorbic acid or salt thereof, or citric acid. A beverage having a pH thus adjusted can be stored long and has an adequate acid taste.

In the packaged effervescent beverage of the present invention, the sweetener (B) comprises erythritol and at least one selected from fruit sugar, grape sugar and high-fructose corn syrup.
These sweeteners are contained in the packaged effervescent beverage of the present invention in an amount of preferably from 0.0001 to 20 mass%, more preferably from 0.001 to 15 mass%, even more preferably from 0.01 to 10 mass%. The packaged effervescent beverage of the present invention containing an unduly small amount of a sweetener can hardly provide sweetness and loses balance between an acid taste and a salty taste so that the intensity of sweetness is preferably 2 or more assuming that the intensity of sweetness of cane sugar is 1 (Reference: JIS Z 8144, Sensory Analysis - Vocabulary, No. 3011, Sweetness: JIS Z 9080, Sensory analysis- Methodology, test method: Dictionary of Beverage Terminology 4-2 Classification of sweetness intensity, Material 11 (Beverage Japan, Inc.); Characteristic grade test mAG test, ISO 6564-1885(E), "Sensory Analysis-Methodology-Flavour Profile Method", etc.). When sweetness is intensified up to 8 or more, on the other hand, such sweetness is felt too strong to be freed from bad feeling at the throat, leading to a lowered throat sensation during its drinking. The above-described sweeteners may also be those contained in the tea extract.

The content of grape sugar in the packaged effervescent beverage of the present invention is preferably from 0.0001 to 20 mass%, more preferably from 0.001 to 15 mass%, even more preferably from 0.01 to 10 mass%. Fructose known as fruit sugar is a ketohexose. The content of grape sugar in the packaged effervescent beverage of the present invention is preferably from 0.0001 to 20 mass%, more preferably from 0.001 to 15 mass%, even more preferably from 0.001 to 10 mass%. The high fructose corn syrup is a mixed syrup of grape sugar and fruit sugar and the content of it is preferably from 0.01 to 7 mass%, more preferably from 0.1 to 6 mass%, even more preferably from 1.0 to 5 mass%. These sweeteners added in a total amount of 20 mass% or more may turn brownish during storage of the beverage and thereby cause coloring of the beverage.

As the sweeteners to be used in the packaged effervescent beverage of the present invention, examples of artificial sweeteners include high-intensity sweeteners such as aspartame, sucralose, saccharin, cyclamate, acesulfame-K, L-aspartyl-L-phenylalanine lower alkyl ester, L-aspartyl-D-alanine amide, L-aspartyl-D-serine amide, L-aspartyl-hydroxymethylalkane amide, L-aspartyl-1-hydroxyethylalkane amide, and sucralose, glycyrrhizin, and synthetic alkoxy aromatic compounds. In addition, thaumatin, stevioside, and other natural origin sweeteners are also usable.

The packaged effervescent beverage of the present invention is characterized in that a carbon dioxide gas (C) contained in the beverage has an adequate foaming property and therefore can reduce the bitterness derived from non-polymer catechins and moreover continuously impart the beverage with soft and cool feel. The carbon dioxide gas injected into the beverage has a gas volume of from more than 0.5 to 4.0 vol.% from the viewpoint of the flavor of the beverage. It provides refreshed feel during drinking. When the gas volume is controlled to 0.5 vol.% or more, the carbon dioxide gas is effective for suppressing bitterness derived from non-polymer catechins. When the gas volume is lowered to 4.0 vol.% or less, on the other hand, stimulation becomes not too strong, so that the fresh feeling of the flavor continues to be preserved.

The term "gas volume" as used herein means a volume ratio of a carbon dioxide gas dissolved in a beverage under 1 atmospheric pressure at 15.6°C to the beverage. An injection operation of a carbon dioxide gas into a beverage composition is called "carbonation" (carbon dioxide injection). Carbonation is typically performed by bringing a carbon dioxide gas into contact with the beverage composition in an apparatus called "carbonator". A carbon dioxide gas is absorbed in a liquid more smoothly at lower temperatures so that carbonation is preferably conducted while cooling to 10°C or less. At a certain temperature, the higher the pressure of a carbon dioxide gas in the carbonater, the higher the absorption efficiency. Carbonation is conducted typically under a pressure of from 0.1 to 4 kg/cm². A carbon dioxide gas is filled at a gas pressure of 3.0 kg/cm² or more for producing Cola or carbonated beverages, at a gas pressure of 0.7 kg/cm² or more for producing beverages containing not a juice but a fruit flavor, and at a pressure of 0.2 kg/cm² or more for producing beverages containing a juice or a milk product.

The packaged effervescent beverage of the present invention may contain sodium and/or potassium, that is, an electrolyte in an amount of from 0.001 to 0.5 mass% and 0.001 to 0.2 mass%, respectively. The total concentration of sodium and potassium is preferably from 0.001 to 0.5 mass%. The total concentrations less than 0.001 mass% are not preferred because a beverage having such a concentration tends to taste as if something is missing, depending on a drinking scene. The total concentrations exceeding 0.5 mass%, on the other hand, are not fit for long-term drinking because of the salty.

As the sodium, a sodium salt such as sodium ascorbate, sodium chloride, sodium carbonate, sodium hydrogen carbonate, sodium citrate, sodium phosphate, sodium hydrogen phosphate, sodium tartrate, or sodium benzoate, or a mixture thereof can be added in the present invention. The sodium may be that derived from the juice or tea component added to the beverage. The higher the sodium concentration, the higher the discoloration degree of the beverage. The sodium content in the packaged effervescent beverage of the present invention is preferably from 0.001 to 0.5 mass%, more preferably from 0.002 to 0.4 mass%, still more preferably from 0.003 to 0.2 mass% from the viewpoint of the stability of the beverage as a product.

The concentration of potassium to be used in the present invention can be heightened by adding a compound other than potassium contained in a tea extract. For example, a potassium salt such as potassium chloride, potassium carbonate, potassium sulfate, potassium acetate, potassium hydrogen carbonate, potassium citrate, potassium phosphate, potassium hydrogen phosphate, potassium tartrate, or potassium sorbate, or a mixture thereof may be added. Potassium may be derived from a juice or flavor added to the beverage. The potassium concentration has a greater influence on the color tone of the beverage stored for a long period at a high temperature compared with the sodium concentration. The potassium content in the packaged effervescent beverage of the present invention is preferably from 0.001 to 0.2 mass%, more preferably from 0.002 to 0.15 mass%, still more preferably from 0.003 to 0.12 mass% from the viewpoint of stability.

The packaged effervescent beverage according to the present invention may contain an acidulant. The beverage having a low acidulant concentration has suppressed bitterness and astringency, but its sourness is too weak. The beverage having a high acidulant concentration has, on the other hand, enhanced sourness, but at the same time has enhanced bitterness or astringency. At least one acidulant selected from ascorbic acid, citric acid, gluconic acid, succinic acid, tartaric acid, lactic acid, fumaric acid, phosphoric acid, and malic acid, and salts thereof may be used in the present invention. Specific examples of it include trisodium citrate, monopotassium citrate, tripotassium citrate, sodium gluconate, potassium gluconate, sodium tartrate, trisodium tartrate, potassium hydrogen tartrate, sodium lactate, potassium lactate, and sodium fumarate.

As another acidulant, adipic acid or salts thereof, and juices extracted from natural components can be used. The acidulants are contained in the packaged effervescent beverage of the present invention in a total amount of preferably from 0.01 to 0.7 mass%, even more preferably from 0.02 to 0.6 mass%. Inorganic salts or salts thereof may also be used as the acidulant. Examples of the inorganic acids or salts thereof include diammonium hydrogen phosphate, ammonium dihydrogen phosphate, dipotassium hydrogen phosphate, disodium hydrogen phosphate, sodium dihydrogen phosphate, trisodium metaphosphate, and tripotassium phosphate. These inorganic acids or salts thereof are contained in the packaged effervescent beverage of the present invention in an amount of preferably from 0.01 to 0.5 mass%, even more preferably from 0.02 to 0.3 mass.

In the packaged effervescent beverage of the present invention, flavors and fruit juices are preferably incorporated to improve its taste. Natural or synthetic flavors and fruit juices may be used in the present invention. They can be selected from fruit juices, fruit flavors, and plant flavors, and mixtures thereof. In particular, combinations of a fruit juice and a tea flavor, preferably green tea or black tea flavor are preferred. Particularly preferred fruit juices usable in the present invention include apple, pear, lemon, lime, mandarin, grapefruit, cranberry, orange, strawberry, grape, kiwi, pineapple, passion fruit, mango, guava, raspberry and cherry juices. Of these, citrus juices such as grapefruit, orange, lemon, lime and mandarin, mango juice, passion fruit juice, and guava juice, and mixtures thereof are even more preferred. Preferred examples of natural flavors include jasmine, chamomile, rose, peppermint, Crataegus cuneata, chrysanthemum, water caltrop, sugarcane, lychee, and bamboo shoot. The juice is contained in the packaged effervescent beverage of the present invention in an amount of preferably from 0.001 to 20 wt.%, more preferably from 0.002 to 10 wt.%. Particularly preferred flavors include citrus flavors such as orange flavor, lemon flavor, lime flavor, and grapefruit flavor. As well as such citrus flavors, various other fruit flavors such as apple flavor, grape flavor, raspberry flavor, cranberry flavor, cherry flavor, and pineapple flavor are also usable. These flavors may be derived from natural sources such as fruit juices and balms, or may be synthesized.
The term "flavor" may embrace blends of various flavors, for example, a blend of lemon and lime flavors and a blend of a citrus flavor and a selected spice (typically, cola soft drink flavor). The flavor may be incorporated in an amount of preferably from 0.0001 to 5 wt.%, more preferably from 0.001 to 3 wt.% in the packaged effervescent beverage of the present invention.

The packaged effervescent beverage of the present invention may further contain vitamins. Preferred vitamins include vitamin A, and vitamin E. Other vitamins such as vitamin D may also be added. Vitamin B is selected from inositol, thiamine hydrochloride, thiamine nitrate, riboflavin, sodium riboflavin-5'-phosphate, niacin, nicotinic acid amide, calcium pantothenate, pyridoxine hydrochloride, and cyanocobalamin, each belonging to the Vitamin B group. Folic acid and biotin are also usable for the beverage of the present invention. The vitamins are added preferably in an amount of at least 10 mass% of a daily requirement per beverage (based on U.S. RDI (U.S. Reference Daily Intake) described in U. S. Patent No. 2005/0003068).

Minerals may also be incorporated in the packaged effervescent beverage of the present invention. Preferred minerals include calcium, chromium, copper, fluorine, iodine, iron, magnesium, manganese, phosphorus, selenium, silicon, molybdenum, and zinc. Of these, magnesium, phosphorus, and iron are highly preferred.

The packaged effervescent beverage of the present invention may contain cyclodextrins in order to suppress the bitterness derived from non-polymer catechins. The cylodextrins include α-cyclodextrin, β-cyclodextrin, and γ-cyclodextrin. These cyclodextrins are incorporated in an amount of preferably from 0.005 to 0.5 mass%, more preferably from 0.02 to 0.3 mass%, especially preferably from 0.05 to 0.25 mass% in the packaged beverage of the present invention.

The packaged effervescent beverage of the present invention may contain, either singly or in combination, additives such as antioxidants, various esters, colorants, emulsifiers, preservatives, seasoning agents, vegetable extracts, flower honey extracts, and quality stabilizers, depending on the components derived from tea.

The packaged effervescent beverage of the present invention may be provided as a tea beverage or a non-tea beverage. Examples of the tea beverage include unfermented tea beverages such as green tea beverage, semi-fermented tea beverages such as Oolong tea beverage, and fermented tea beverages such as black tea beverage. The packaged effervescent beverage of the present invention may be provided as a functional beverage. For example, it may be provided as a non-tea beverage such as enhanced water, bottled water, sports drink or near water.

The calorie of the packaged effervescent beverage of the present invention is calculated assuming that the calorie of each of grape sugar, fruit sugar, and cane sugar contained in 100 ml of the beverage is 4 kcal/g and that of erythritol is 0 kcal/g. The packaged effervescent beverage of the present invention is a low caloric beverage having a calorie of 40 kcal/240 ml or less, more preferably from 2 to 35 kcal/240 ml or less, even more preferably from 3 to 30 kcal/240 ml or less.

The packaged effervescent beverage of the present invention can be provided as a beverage packed in a conventional package such as a molded package having polyethylene terephthalate as a principal component (a so-called PET bottle), a metal can, a paper container combined with a metal foil or plastic film, or a bottle. The term "packaged effervescent beverage" as used herein means a beverage that can be consumed without dilution.

The packaged effervescent beverage of the present invention may be produced by the process conventionally employed for the production of carbonated beverages. The production process can be classified into a post mixing system and a premixing system. The former one is to prepare a mixed solution of non-polymer catechins, a sweetener, and water, add a juice, a flavor, an acidulant and the like to the resulting mixed solution to prepare a beverage composition, pour a predetermined amount of the beverage composition into a package such as PET bottle, and then fill the resulting package with carbonated water. The latter one is to mix non-polymer catechins, a sweetener, a juice, and water, which have been added at a predetermined ratio, in a volumetric mixer, injecting a carbon dioxide gas into the resulting mixture while cooling it, and then fill a package such as PET bottle with the resulting beverage composition. The beverage composition thus filled in the package is thermally sterilized under sterilization conditions prescribed in the Food Sanitation Act, preferably at a temperature of from 59 to 62°C for from about 10 to 15 minutes to sterilize the beverage contained in the package.

### [Examples]

### Measurement of Non-polymer Catechins

A non-polymer catechin content of a sample which has been filtered through a membrane filter (0.8 µm) and then diluted with distilled water, is measured by the gradient elution method using high-performance liquid chromatograph ("SCL-10AVP", product of Shimadzu Corporation) equipped with "L-Column TM ODS" (packed column for octadecyl-introduced liquid chromatograph, 4.6 mmφ × 250 mm: product of Chemicals Evaluation and Research Institute, Japan) at a column temperature of 35°C. Measurement is conducted under the following conditions: use of a solution of 0.1 mol/L of acetic acid in distilled water as a mobile phase solution A and a solution of 0.1 mol/L of acetic acid in acetonitrile as a mobile phase solution B, an injection amount of a sample: 20 µL, and a wavelength of UV detector at 280 nm (the catechin concentration and caffeine concentration are usually expressed by mass/vol.% (%[w/v]) but the content in Examples is expressed by mass obtained by multiplying it by the liquid amount).

### Measurement of gas volume

The gas volume is measured using Gas Volume/Air Meter Model GVA-500 (Kyoto Electronics Manufacturing Co., Ltd.)

### Evaluation of taste

A drinking test is performed by a panel of five experts. Evaluation standards are shown in the column below Table 1 and Table 2.

### Storage Test

The beverage thus prepared is stored at 37°C for 4 weeks and a change in the color tone of the beverage before and after storage is visually observed by a panel of five experts. The beverage is rated in accordance with the standards described in the column below Table 1 and Table 2. In addition, a non-polymer catechin content after storage is determined.

### Example 1

A purified product was obtained by dispersing 100 g of a commercially-available purified product of green tea extract ("Polyphenone HG", product of Mitsui Norin) in 900 g of 90.0 mass% ethanol, aging the resulting dispersion for 30 minutes, filtering it through a No. 2 filter paper and a filter paper with a pore size of 0.2 µm, adding 200 mL of water to the filtrate, and then concentrating the resulting mixture under reduced pressure. The purified product thus obtained contained 15.2 mass% of non-polymer catechins (A) and the percentage of non-polymer catechin gallates (E) was 58.1 mass%. A 75.0 g portion of the non-polymer catechin composition thus obtained was poured in a stainless vessel and ion exchanged water was added to give a total amount of 1,000 g. Then, 3.0 g of a 5 mass% aqueous sodium bicarbonate solution was added to adjust the pH to 5.5. A solution obtained by dissolving 0.27 g (2.4% relative to the non-polymer catechins) of Kikkoman tannase KTFH (Industrial Grade, 500 U/g or more) in 1.07 g of ion exchanged water under stirring conditions at 22°C and 150 r/min was then added. At the time point when the pH reduced to 4.24 after 55 minutes, the enzyme reaction was terminated. The stainless vessel was then immersed in a hot bath of 95°C. After complete deactivation of the enzyme activity by retaining the vessel at 90°C for 10 minutes and then cooling to 25°C, concentration treatment was performed. The purified product of green tea extract obtained after tannase treatment contained 15.0 mass% of non-polymer catechins and the non-polymer gallate percentage was 45.2 mass%. To 8.5 g of the resulting purified product of green tea extract were added 36.6 g of anhydrous crystalline fructose, 7.5 g of erythritol, 1.0 g of anhydrous citric acid, 1.2 g of trisodium citrate, 0.5 g of L-ascorbic acid, and 1.0 g of a lemon lime flavor. Carbonated water cooled to 2°C was then added to give a total amount to 1,000 g (carbon dioxide gas: 2.2 vol.%). A pressure bottle made of PET was filled with 500 g of the resulting mixture. Then, the mixture in the bottle was heat sterilized at 60°C for 12 minutes. The composition, taste evaluation results, and storage test results of the packaged effervescent beverage, are shown in Table 1.

### Examples 2 to 5

A grape fruit beverage and an orange beverage were produced in a similar manner to Example 1 except that the corresponding flavors were used, respectively, instead of the lemon lime flavor. In addition, a cola beverage and a root beer beverage were produced in a similar manner except that a carbon dioxide gas volume was changed to 3.5 vol.%.

### Example 6

In a similar manner to Example 1 except that 1.0 g of a lemon lime juice was added and the anhydrous crystalline fructose was replaced by 36.6 g of anhydrous crystalline glucose, a beverage was produced.

### Comparative Example 1

In a similar manner to Example 1 except that the amount of trisodium citrate was increased to 15.0 g, a beverage was produced.

### Comparative Example 2

In a similar manner to Example 1 except for the omission of the anhydrous crystalline fructose and erythritol, a beverage was produced.

### Comparative Example 3

In a similar manner to Example 1 except for the omission of the carbon dioxide gas, a beverage was produced.

### Comparative Example 4

In a similar manner to Example 1 except that a carbon dioxide gas volume was changed to 5.0 vol.%, a beverage was produced.

### Example 7

After addition of 5.3 g of the purified product of green tea extract obtained in Example 1, 2.2 g of Chinese green-tea extract powder, 36.6 g of anhydrous crystalline fructose, 7.5 g of erythritol, 0.5 g of L-ascorbic acid, and 1.0 g of a green tea flavor, carbonated water cooled to 2°C was added to the resulting mixture to give a total amount of 1,000 g (a carbon dioxide gas: 2.2 vol.%). The resulting mixture was treated in a similar manner to Example 1 to obtain a packaged effervescent beverage. The composition, taste evaluation results, and storage test results of the resulting beverage are shown in Table 2.

### Example 8

After addition of 8.5 g of the purified product of green tea extract obtained in Example 1, 0.5 g of Indian black-tea extract powder, 36.6 g of anhydrous crystalline fructose, 7.5 g of erythritol, 0.5 g of L-ascorbic acid, 2.0 g of a 5% aqueous sodium bicarbonate solution, and 1.0 g of a black tea flavor, carbonated water cooled to 2°C was added to give a total amount of 1,000 g (carbon dioxide gas: 2.2 vol.%). The resulting mixture was treated in a similar manner to Example 1 to obtain a packaged effervescent beverage. The composition, taste evaluation results, and storage test results of the resulting beverage are shown in Table 2.

### Comparative Example 5

In a similar manner to Example 7 except that L-ascorbic acid was replaced by 0.3 g of sodium L-ascorbate and 1.5 g of a 5% aqueous sodium bicarbonate solution was added, a beverage was produced.

### Comparative Example 6

In a similar manner to Example 7 except for the omission of anhydrous crystalline fructose and erythritol, a beverage was produced.

Tables 1 and 2 have revealed that packaged effervescent beverages having a high concentration of non-polymer catechins, a sweetener, and a carbon dioxide gas have remarkably reduced bitterness and can be stored for a long term.

**[Table 1]**

| | | Example 1 | Example 2 | Example 3 | Example 4 | Example 5 | Example 6 | Comp. Ex. 1 | Comp. Ex. 2 | Comp. Ex. 3 | Comp. Ex. 4 |
|---|---|---|---|---|---|---|---|---|---|---|---|
| | Purified product of green tea extract containing non-polymer catechins (mass%) | 0.85 | 0.85 | 0.85 | 0.85 | 0.85 | 0.85 | 0.85 | 0.85 | 0.85 | 0.85 |
| | Anhydrous crystalline glucose (mass%) | - | - | - | - | - | 3.66 | - | - | - | - |
| | Anhydrous crystalline fructose (mass%) | 3.66 | 3.66 | 3.66 | 3.66 | 3.66 | - | 3.66 | - | 3.66 | 3.66 |
| | Erythritol (mass%) | 0.75 | 0.75 | 0.75 | 0.75 | 0.75 | 0.75 | 0.75 | - | 0.75 | 0.75 |
| | Anhydrous citric acid (mass%) | 0.1 | 0.1 | 0.1 | 0.1 | 0.1 | 0.1 | 0.1 | 0.1 | 0.1 | 0.1 |
| | 3Na Citrate (mass%) | 0.12 | 0.12 | 0.12 | 0.12 | 0.12 | 0.12 | 1.5 | 0.12 | 0.12 | 0.12 |
| | L-Ascorbic acid (mass%) | 0.05 | 0.05 | 0.05 | 0.05 | 0.05 | 0.05 | 0.05 | 0.05 | 0.05 | 0.05 |
| Composition | Lemon lime flavor (mass%) | 0.1 | - | - | - | - | 0.1 | 0.1 | 0.1 | 0.1 | 0.1 |
| | Lemon lime juice (mass%) | - | - | - | - | - | 0.1 | - | - | - | |
| | Grape fruit flavor (mass%) | - | 0.1 | - | - | - | - | - | - | - | - |
| | Orange flavor (mass%) | - | - | 0.1 | - | - | - | - | - | - | - |
| | Cola extract (mass%) | - | - | - | 0.1- | - | - | - | - | - | - |
| | Root beer extract (mass%) | - | - | - | - | 0.1 | - | - | - | - | - |
| | Caramel colorant (mass%) | - | - | - | 0.3 | 0.3 | - | - | - | - | - |
| | Carbonated water (mass%) | Balance | Balance | Balance | Balance | Balance | Balance | Balance | Balance | Balance | Balance |
| | Total amount (mass%) | 100 | 100 | 100 | 100 | 100 | 100 | 100 | 100 | 100 | 100 |
| (B) Sweetener (mass%) | | 4.41 | 4.41 | 4.41 | 4.41 | 4.41 | 4.41 | 4.41 | 0 | 4.41 | 4.41 |
| (C) Carbon dioxide gas (vol.%) | | 2.2 | 2.2 | 2.2 | 3.5 | 3.5 | 2.2 | 2.2 | 2.2 | 0 | 5.0 |
| | (A) Non-polymer catechins (mass%) | 0.128 | 0.128 | 0.128 | 0.128 | 0.128 | 0.128 | 0.128 | 0.128 | 0.128 | 0.128 |
| | (B) Non-epicatechin percentage (mass%) | 15.0 | 15.0 | 15.0 | 15.0 | 15.0 | 15.0 | 30.2 | 15.0 | 15.0 | 15.0 |
| | (F) pH | 3.98 | 3.96 | 4.03 | 4.00 | 4.02 | 4.01 | 6.01 | 4.00 | 3.98 | 3.98 |
| | (E) Gallate percentage (mass%) | 45.2 | 45.2 | 45.2 | 45.2 | 45.2 | 45.2 | 45.2 | 45.2 | 45.2 | 45.2 |
| After | (G) Caffeine/(A) | 0.052 | 0.052 | 0.052 | 0.052 | 0.052 | 0.052 | 0.052 | 0.052 | 0.052 | 0.052 |
| sterilization | Evaluation of bitterness¹⁾ | 1 | 1 | 1 | 1 | 1 | 1 | 1 | 5 | 3 | 3 |
| | Evaluation of stimulating taste ³⁾ | 2 | 2 | 2 | 2 | 2 | 2 | 2 | 2 | 1 | 3 |
| | Color tone after storage (37°C. 4 weeks) | A | A | A | A | A | A | C | A | A | A |
| | Non-polymer catechins after storage (37°C, 4 weeks) (mass%) | 0.120 | 0.118 | 0.121 | 0.120 | 0.116 | 0.117 | 0.087 | 0.123 | 0.120 | 0.120 |

| | | | | | | | | | | | |
|---|---|---|---|---|---|---|---|---|---|---|---|
| 1) Five-grade evaluation of bitterness, 1: having no bitterness, 2: having greatly reduced bitterness, 3: having reduced bitterness, 4: having slightly reduced bitterness, 5: having bitterness 2) Three-grade evaluation of a taste providing a cool refreshing feeling, 1: having no cool refreshing feeling, 2: having an adequate cool refreshing feeling, 3: too stimulating. 3) Color tone after storage, A: no change, B: slight change, C: moderate change, D: great change | | | | | | | | | | | |

**[Table 2]**

| | | Example 7 | Example 8 | Comp. Ex. 5 | Comp. Ex. 6 |
|---|---|---|---|---|---|
| Composition | Purified product of green tea extract containing non-polymer catechins (mass%) | 0.53 | 0.85 | 0.53 | 0.53 |
| | Green tea extract (mass%) | 0.22 | - | 0.22- | 0.22- |
| | Black tea extract (mass%) | - | 0.05 | - | - |
| | Anhydrous crystalline fructose (mass%) | 3.66 | 3.66 | 3.66 | - |
| | Erythritol (mass%) | 0.75 | 0.75 | 0.75 | - |
| | L-ascorbic acid (mass%) | 0.05 | 0.05 | - | 0.05 |
| | Na L-ascorbate (mass%) | - | - | 0.03 | - |
| | 5% Aqueous sodium bicarbonate solution (mass%) | - | 0.2 | 0.15 | - |
| | Green tea flavor (mass%) | 0.1 | | 0.1 | 0.1 |
| | Black tea flavor (mass%) | - | 0.1 | - | - |
| | Carbonated water (mass%) | Balance | Balance | Balance | Balance |
| | Total amount (mass%) | 100 | 100 | 100 | 100 |
| (B) Sweetener (mass%) | | 4.41 | 4.41 | 4.41 | 0 |
| (C) Carbon dioxide gas (vol.%) | | 2.2 | 2.2 | 2.2 | 2.2 |
| After sterilization | (A) Non-polymer catechins (mass%) | 0.134 | 0.129 | 0.134 | 0.134 |
| | (B) Non-epicatechin percentage (mass%) | 15.1 | 15.1 | 30.3 | 15.1 |
| | (F) pH | 4.01 | 3.99 | 5.99 | 4.00 |
| | (E) Gallate percentage (mass%) | 50.3 | 45.2 | 50.3 | 50.3 |
| | (G) Caffeine/(A) | 0.104 | 0.079 | 0.104 | 0.104 |
| | Evaluation of bitterness ¹⁾ | 1 | 1 | 1 | 5 |
| | Evaluation of stimulating property ²⁾ | 2 | 2 | 2 | 2 |
| Color tone after storage (37°C, 4 weeks) ³⁾ | | A | A | C | A |
| Non-polymer catechins after storage (37°C, 4 weeks) (mass%) | | 0.129 | 0.123 | 0.098 | 0.128 |

| | | | | | |
|---|---|---|---|---|---|
| 1) Five-grade evaluation of bitterness, 1: having no bitterness, 2: having greatly reduced bitterness, 3: having reduced bitterness, 4: having slightly reduced bitterness, 5: having bitterness 2) Three-grade evaluation of a taste providing a cool refreshing feeling, 1: having a weak cool refreshing feeling, 2: having an adequate cool refreshing feeling, 3: too stimulating. 3) Color tone after storage, A: no change, B: slight change, C: moderate change, D: great change | | | | | |

## Claims

1. A packaged effervescent beverage with a calorie less than 40 Kcal/240 ml, having a purified product of green tea extract added thereto, comprising:
(A) from 0.08 to 0.5 mass% of non-polymer catechins,
(B) from 0.0001 to 20 mass% of (B) a sweetener, and
(C) a carbon dioxide gas, whose gas volume ranges from more than 0.5 vol.% to 4.0 vol.%,
wherein:
(D) a non-epicatechin percentage in the non-polymer catechins is from 5 to 25 mass%,
a percentage of (E) gallates in (A) the non-polymer catechins ([(E)/(A)] × 100) is from 5 to 55 mass%,
(F) the beverage has a pH of from 2.5 to 5.1; and
a mass ratio of (G) caffeine to (A) the non-polymer catechins [(G)/(A)] is from 0.0001 to 0.16; and
wherein the sweetener (B) comprises erythritol and at least one selected from fruit sugar, grape sugar and high-fructose corn syrup.

2. The packaged effervescent beverage according to Claim 1, wherein the sweetener comprises an artificial sweetener.

3. The packaged effervescent beverage according to Claim 1 or 2, further comprising at least one acidulant selected from ascorbic acid, citric acid, gluconic acid, succinic acid, tartaric acid, lactic acid, fumaric acid, phosphoric acid, and malic acid, and salts thereof.

4. The packaged effervescent beverage according to any one of Claims 1 to 3, further comprising at least one mineral selected from sodium, potassium, calcium, magnesium, zinc, and iron.

5. The packaged effervescent beverage according to any one of Claims 1 to 4, further comprising at least one Vitamin B selected from inositol, thiamine hydrochloride, thiamine nitrate, riboflavin, sodium riboflavin-5'-phosphate, niacin, nicotinic acid amide, calcium pantothenate, pyridoxine hydrochloride, cyanocobalamin, folic acid and biotin in an amount of at least 10 mass% of a daily requirement per beverage.

6. The packaged effervescent beverage according to any one of Claims 1 to 5, which has a sweetness intensity of 2 or more assuming that the sweetness intensity of cane sugar is 1.

7. The packaged effervescent beverage according to any one of Claims 1 to 6, which is a tea beverage.

8. The packaged effervescent beverage according to any one of Claims 1 to 7, which is a non-tea beverage.

9. The packaged effervescent beverage according to any one of Claims 1 to 8, further comprising at least one selected from juices, vegetable juices, and flavors.

10. The packaged effervescent beverage according to any one of Claims 1 to 9, further comprising a milk component.

11. The packaged effervescent beverage according to any one of Claims 8 to 10, which is enhanced water.

## Patentansprüche

1. Verpacktes kohlensäurehaltiges Getränk mit einem Brennwert von weniger als 40 Kcal/240 ml, dem ein gereinigtes Erzeugnis aus Grüntee-Extrakt hinzugefügt ist, umfassend:
(A) 0,08 bis 0,5 Massen% nicht-polymerer Catechine,
(B) 0,0001 bis 20 Massen% eines Süßmittels (B), und
(C) Kohlendioxidgas, dessen Gasvolumen im Bereich von mehr als 0,5 Vol.% bis 4,0 Vol.% ist,
worin
(D) der Prozentsatz an Nicht-Epicatechin in den nicht-polymeren Catechinen von 5 bis 25 Massen% ist,
ein Prozentsatz von (E) Gallaten in den nicht-polymeren Catechinen (A) ([(E)/(A)] x 100) von 5 bis 55 Massen% ist,
(F) das Getränk einen pH-Wert von 2,5 bis 5,1 hat; und
ein Massenverhältnis von Koffein (G) zu den nicht-polymeren Catechinen (A) [(G)/(A)] von 0,0001 bis 0,16 ist; und
worin das Süßmittel (B) Erythritol und mindestens eines ausgewählt aus Fruchtzucker, Traubenzucker und Maissirup mit hohem Fruktosegehalt umfasst.

2. Verpacktes kohlensäurehaltiges Getränk gemäß Anspruch 1, worin das Süßmittel ein künstliches Süßmittel umfasst.

3. Verpacktes kohlensäurehaltiges Getränk gemäß Anspruch 1 oder 2, das weiterhin mindestens eine Säuerungsmittel ausgewählt aus Ascorbinsäure, Zitronensäure, Glukonsäure, Bernsteinsäure, Weinsäure, Milchsäure, Fumarsäure, Phosphorsäure und Äpfelsäure und Salze davon umfasst.

4. Verpacktes kohlensäurehaltiges Getränk gemäß irgendeinem der Ansprüche 1 bis 3, das weiterhin mindestens einen Mineralstoff ausgewählt aus Natrium, Kalium, Kalzium, Magnesium, Zink und Eisen umfasst.

5. Verpacktes kohlensäurehaltiges Getränk gemäß irgendeinem der Ansprüche 1 bis 4, das weiterhin mindestens einen Vitamin B ausgewählt aus Inositol, Thiaminhydrochorid, Thiaminnitrat, Riboflavin, Natriumriboflavin-5'-Phosphat, Niacin, Nikotinsäureamid, Kalziumpantothenat, Pyridoxinhydrochlorid, Cyanocobalamin, Folsäure und Biotin in einer Menge von mindestens 10 Massen% der benötigten Tagesdosis pro Getränk umfasst.

6. Verpacktes kohlensäurehaltiges Getränk gemäß irgendeinem der Ansprüche 1 bis 5, das eine Intensität der Süße von 2 oder mehr aufweist, unter der Annahme, dass die Intensität der Süße von Rohrzucker 1 ist.

7. Verpacktes kohlensäurehaltiges Getränk gemäß irgendeinem der Ansprüche 1 bis 6, das ein Teegetränkt ist.

8. Verpacktes kohlensäurehaltiges Getränk gemäß irgendeinem der Ansprüche 1 bis 7, das ein Nicht-Teegetränkt ist.

9. Verpacktes kohlensäurehaltiges Getränk gemäß irgendeinem der Ansprüche 1 bis 8, das weiterhin mindestens eines ausgewählt aus Säften, Gemüsesäften und Geschmacksstoffen umfasst.

10. Verpacktes kohlensäurehaltiges Getränk gemäß irgendeinem der Ansprüche 1 bis 9, das weiterhin eine Milchkomponente umfasst.

11. Verpacktes kohlensäurehaltiges Getränk gemäß irgendeinem der Ansprüche 8 bis 10, das ein verbessertes (enhanced) Wasser ist.

## Revendications

1. Boisson effervescente conditionnée ayant une valeur énergétique inférieure à 40 Kcal/240 mL, à laquelle est ajouté un produit purifié d'extrait de thé vert, comprenant :
(A) de 0,08 à 0,5 % en masse de catéchines non polymères,
(B) de 0,0001 à 20 % en masse de (B) un édulcorant, et
(C) un gaz dioxyde de carbone, dont le volume de gaz se trouve dans la plage de plus de 0,5 % en volume à 4,0 % en volume,
dans laquelle :
(D) un pourcentage de non-épicatéchine dans les catéchines non polymères est de 5 à 25 % en masse,
un pourcentage des (E) gallates dans (A) les catéchines non polymères ([(E)/(A)] x 100) est de 5 à 55 % en masse,
(F) la boisson a un pH de 2,5 à 5,1 ; et
un rapport en masse de (G) la caféine sur (A) les catéchines non polymères [(G)/(A)] est de 0,0001 à 0,16 ; et
dans lequel l'édulcorant (B) comprend de l'érythritol et au moins un sélectionné parmi un sucre de fruit, un sucre de raisin et un sirop de glucose à haute teneur en fructose.

2. Boisson effervescente conditionnée selon la revendication 1, dans laquelle l'édulcorant comprend un édulcorant artificiel.

3. Boisson effervescente conditionnée selon la revendication 1 ou 2, comprenant en outre au moins un acidulant sélectionné parmi l'acide ascorbique, l'acide citrique, l'acide gluconique, l'acide succinique, l'acide tartrique, l'acide lactique, l'acide fumarique, l'acide phosphorique, et l'acide malique, et leurs sels.

4. Boisson effervescente conditionnée selon l'une quelconque des revendications 1 à 3, comprenant en outre au moins un minéral sélectionné parmi le sodium, le potassium, le calcium, le magnésium, le zinc, et le fer.

5. Boisson effervescente conditionnée selon l'une quelconque des revendications 1 à 4, comprenant en outre au moins une vitamine B sélectionnée parmi l'inositol, le chlorhydrate de thiamine, le nitrate de thiamine, la riboflavine, le riboflavin-5'-phosphate de sodium, la niacine, l'amide de l'acide nicotinique, le pantothénate de calcium, le chlorhydrate de pyridoxine, la cyanocobalamine, l'acide folique et la biotine en une quantité d'au moins 10 % en masse des besoins journaliers par boisson.

6. Boisson effervescente conditionnée selon l'une quelconque des revendications 1 à 5, qui a une intensité de sucrosité de 2 ou plus en supposant que l'intensité de sucrosité du sucre de canne est 1.

7. Boisson effervescente conditionnée selon l'une quelconque des revendications 1 à 6, qui est une boisson à base de thé.

8. Boisson effervescente conditionnée selon l'une quelconque des revendications 1 à 7, qui est une boisson autre que le thé.

9. Boisson effervescente conditionnée selon l'une quelconque des revendications 1 à 8, comprenant en outre au moins l'un choisi parmi des jus, des jus de légumes, et des arômes.

10. Boisson effervescente conditionnée selon l'une quelconque des revendications 1 à 9, comprenant en outre un composant lacté.

11. Boisson effervescente conditionnée selon l'une quelconque des revendications 8 à 10, qui est de l'eau renforcée.
